# EUROPÄISCHE PATENTSCHRIFT

(11) **EP 2 448 892 B1**
(45) Veröffentlichungstag und Bekanntmachung des Hinweises auf die Patenterteilung: **10.07.2013**
(21) Anmeldenummer: 10720622.9
(22) Anmeldetag: 21.05.2010
(51) Int. Cl.: C07C 7/10, C07C 7/163, C07C 9/10

(54) **VERFAHREN ZUR HERSTELLUNG VON GERUCHSARMEM N-BUTAN**
METHOD FOR PRODUCING LOW-ODOR N-BUTANE
PROCÉDÉ DE PRODUCTION DE N-BUTANE PEU ODORANT

(30) Priorität: 01.07.2009 DE 102009027406
(43) Veröffentlichungstag der Anmeldung: 09.05.2012
(73) Patentinhaber: Evonik Oxeno GmbH, 45772 Marl (DE)
(72) Erfinder: LÜKEN, Hans-Gerd, 45770 Marl (DE); KAIZIK, Alfred, 45772 Marl (DE); WINTERBERG, Markus, 45711 Datteln (DE); BÜSCHKEN, Wilfried, 45721 Haltern am See (DE); FRIDAG, Dirk, 45721 Haltern am See (DE)
(86) Internationale Anmeldenummer: PCT/EP2010/057031
(87) Internationale Veröffentlichungsnummer: WO 2011/000633

(56) Entgegenhaltungen:
- WO-A1-02/00582
- WO-A1-99/26937
- DE-A1-102007 061 649
- DE-A1-102008 002 187

## Beschreibung

Die vorliegende Erfindung betrifft ein Verfahren zur Herstellung von geruchsarmem n-Butan durch katalytische Hydrierung eines Einsatzgemisches.

Gerucharmes n-Butan findet Verwendung als Aerosoltreibmittel. Für diese Anwendung darf das n-Butan keine geruchlich störenden Begleitstoffe aufweisen. Außer dem schwachen Eigengeruch des n-Butans darf kein weiterer Geruch wahrnehmbar sein.

Geruchsarmes n-Butan kann durch Hydrierung von n-Butan/n-Buten-Gemischen, die Spuren von mehrfach ungesättigten Kohlenwasserstoffen, wie Butadien, enthalten und/oder geringe Mengen an C₅-Kohlenwasserstoffen aufweisen können, hergestellt werden. Als Katalysatoren für die Hydrierung werden vorzugsweise EdelmetallTrägerkatalysatoren, wie z. B. Palladium auf Aluminiumoxid-Träger, eingesetzt.

Solche Einsatzströme könnten beispielweise Raffinat II oder Raffinat III sein. Aus wirtschaftlichen Gründen werden jedoch bevorzugt Gemische linearer C₄-Kohlenwasserstoffströme verwendet, die hauptsächlich aus n-Butan bestehen.

Ein für die Herstellung von gerucharmem n-Butan verwendetes Einsatzgemisch ist der Reststrom, der bei der Oligomerisierung der linearen Butene von Raffinat II oder Raffinat III nach Abtrennung des Oligomerisats übrig bleibt. Dieser Reststrom besteht einzig aus Kohlenwasserstoffen.

Wird dagegen ein Gemisch linearer C₄-Kohlenwasserstoffe, beispielsweise Raffinat II oder Raffinat III, hydroformyliert, wird nach Abtrennung von Synthesegas und Hydroformylierungsprodukte, ein n-Butan-reiches Gemisch linearer C₄-Kohlenwasserstoffe enthalten, das geringe Mengen an Ameisensäure, C₅-Aldehyde und gegebenenfalls C₅-Alkohole, gegebenenfalls Synthesegas und Wasser aufweist.

Aus folgenden Gründen kann aus solchen Gemischen durch Hydrierung und gegebenenfalls Destillation nur mit hohem Aufwand geruchsarmes n-Butan gewonnen werden: Aus der Ameisensäure entsteht am Hydrierkatalysator Kohlenmonoxid, das dessen Hydrierleistung beeinträchtig, sodass eine Hydrierung von Olefinen im Spurenbereich nur durch geringe Katalysatorbelastungen erreicht werden können. Pentanale werden zum größten zu Pentanolen hydriert. Sowohl Pentanole als auch Pentanale, bereits im Spurenbereich, sind für n-Butan für Aerosolanwendungen nicht zulässig.

Darüber hinaus ist Ameisensäure bereits im Spurenbereich sehr korrosiv, so dass einer Verwendung von preiswerten unlegierten Werkstoffen für die Apparate nicht möglich ist. Aufgrund der korrosiven Wirkung der Ameisensäure steigen die Investitionskosten, da hochlegierte Stähle verwendet werden müssen.

Aus der WO 99/26937 ist es bekannt, Ameisensäure aus einem KohlenwasserstoffGemisch mit Hilfe von Natronlauge auszuwaschen.

Im Lichte dieses Standes der Technik liegt der vorliegenden Erfindung die Aufgabe zu Grunde, ein Verfahren zur Herstellung von geruchsarmen n-Butan durch katalytische Hydrierung eines Einsatzstoffes anzugeben, wobei der Einsatzstoff neben n-Butan, n-Buten sowie bis zu 1 Massen-% Ameisensäure und/oder bis zu 1 Massen-% Pentanale und/oder bis zu 0,5 Massen-% Pentanole auch zusätzlich Kohlenmonoxid enthält.

Gelöst wird diese Aufgabe durch ein Verfahren nach Anspruch 1. Bevorzugte Weiterbildungen der-Erfindung sind in den Unteransprüchen niedergelegt.

Die Erfindung beruht mithin auf der Erkenntnis, dass aus einem kohlenmonoxidhaltigen n-Butan/n-Buten-Gemisch, das geringe Mengen an Ameisensäure und/oder Pentanale und/oder Pentanole aufweist, durch katalytische Hydrierung mit Wasserstoff ein geruchsarmes n-Butan dann hergestellt werden kann, wenn das Einsatzgemisch vor der Hydrierung unter Einhaltung der anspruchsgemäßen Reaktionsparameter mit einer wässrigen Lösung eines Alkalimetall- oder Erdalkalimetallhydroxids behandelt wird. Bei der Behandlung des C₄-Stroms mit Lauge wird die Ameisensäure als Salz gebunden. Pentanale bilden Aldoladdition- bzw. Aldolkondensationsprodukte. Pentanole bleiben in der Lauge gelöst. Erfindungsgemäße Behandlung mit Lauge entfernt zudem auch Kohlenmonoxid aus dem Einsatzgemisch, sodass eine Schädigung des Katalysators bei anschließender Hydrierung vermieden wird.

Das erfindungsgemäße Verfahren auch folgende Vorteile auf: Der Kapitaleinsatz für eine Apparatur zur Abtrennung der störenden Komponenten aus dem n-Butan/n-Buten-Gemisch ist gering. Die Betriebskosten für diese Abtrennung sind klein. Durch die Abtrennung der Ameisensäure werden die Kosten für den Hydrierkatalysator verringert. Darüber hinaus wird die Gefahr der Korrosion durch Ameisensäure verringert, sodass für den Bau der der Laugewäsche nachgeschalteten Anlagen, wie z. B. Destillationskolonnen, Wärmetauscher und Hydrieranlage, preiswerte Stähle verwendet werden können.

Das gesamte Verfahren zur Herstellung zur Herstellung des geruchsarmen n-Butans besteht aus der Abfolge von vier Verfahrensschritten, nämlich Basenextraktion, Entwässerung durch Destillation, Hydrierung der Olefine und optional destillative Abtrennung von C₅-Kohlenwasserstoffen. Der Gegenstand der vorliegenden Erfindung betrifft die Basenextraktion Die Reihenfolge der anderen drei Schritte kann optional abgeändert werden.

Im Folgenden wird die vorliegende Erfindung ausführlich beschrieben.

Einsatzstoffe für das erfindungsgemäße Verfahren sind kohlenmonoxidhaltige Gemische aus n-Butan und linearen Butenen, die als Nebenkomponente(n) Ameisensäure und/oder Pentanal(e) und/oder Pentanole aufweisen. Der Gehalt an linearen Butenen in diesen Gemischen liegt bevorzugt unter 30 Massen-%, insbesondere unter 15 Massen-%, ganz besonders unter 10 Massen-%. Darüber hinaus können diese Gemische mehrfach ungesättigte Kohlenwasserstoffe, wie beispielsweise 1,3-Butadien, und bis zu 1,5 Massen-% C₅-Kohlenwasserstoffe aufweisen. Der Gehalt an Ameisensäure liegt im Bereich von 0,001.bis 1 Massen-%, insbesondere im Bereich von 0,001 bis 0,1 Massen-%, ganz besonders im Bereich von 0,005 bis 0,010Massen-%. Der Gehalt an Pentanalen beträgt 0,001 bis 1 Massen-%, insbesondere 0,001 bis 0,5 Massen-%, ganz besonders bei 0,005 bis 0,010 Massen-%. Der Bereich von Pentanolen liegt im Bereich von 0,001 bis 0,5 Massen-%, insbesondere im Bereich von 0,001 bis 0,2 Massen-%, ganz besonders im Bereich von 0,01 bis 0,1 Massen-%. Der Gehalt an Kohlenmonoxid am Einsatzgemisch liegt im Bereich von 1 bis 10 Massen-%. Darüber hinaus kann das Einsatzgemisch molekularen Wasserstoff enthalten. Das Einsatzgemisch kann einphasig oder zweiphasig sein, etwa mit einer flüssigen Phase und einer Gasphase. Das Kohlenmonoxid kann dabei gasförmig und/oder zumindest teilweise in der flüssigen Phase gelöst sein. Von der Gesamtmenge des Kohlemonoxids liegt über 90 % gasförmig vor.

Typische Einsatzstoffe für das erfindungsgemäße Verfahren sind n-Butan/n-Buten-Gemische, die bei der Aufarbeitung von Produktausträgen einer Hydroformylierung von linearen Butenen anfallen. Solch ein Verfahren wird beispielsweise in DE 10 2008 002187.3 beschrieben. In diesem Verfahren wird ein n-Buten/n-Butan-Gemisch hydroformyliert. Die Produkte und nicht umgesetztes Edukt werden mit überschüssigem Synthesegas aus dem Reaktor getragen. Dieser Gasstrom wird in Synthesegas, Produkte (Pentanale und Folgeprodukte) und in ein n-Butan/n-Butengemisch getrennt. Dabei kann je nach Art der destillativen Aufarbeitung ein gasförmiges, flüssiges oder sowohl ein gasförmiges als auch ein flüssiges Produktgemisch abgetrennt werden.

Bei der destillativen Trennung im Druckbereich von 0,5 MPa bis 3,0 MPa fällt vorzugsweise sowohl ein flüssiger Produktstrom, in dem Synthesegas gelöst ist, als auch ein Synthesegasstrom mit hohem Anteil von C₄-Verbindungen an.

Sowohl der flüssige als auch der gasförmige Strom können getrennt oder gemeinsam nach dem erfindungsgemäßen Verfahren aufgearbeitet werden.

Aus den n-Butan/n-Buten-Gemischen werden erfindungsgemäß störende Komponenten, wie Ameisensäure und/oder Pentanale und/oder Pentanole, durch Behandlung mit einer wässrigen Lauge entfernt.

Als Laugen können wässrige Lösungen von Alkalimetallhydroxid oder Erdalkalimetallhydroxid verwendet werden. Bevorzugt werden Natronlauge und Kalilauge, insbesondere Natronlauge eingesetzt.

Die Konzentration der Hydroxide in der wässrigen Lösung beträgt 0,5 bis 30 Massen-%, insbesondere 2 bis 5 Massen-%. Bei Natronlauge liegt der Natriumhydroxid-Gehalt vorzugsweise im Bereich von 0,5 bis 25 Massen-%, insbesondere im Bereich von 1 bis 5 Massen-%, ganz besonders im Bereich von 2,5 bis 3,5 Massen-%.

Bei der Behandlung mit Lauge wird die im Einsatzgemisch vorhandene Ameisensäure zu dem entsprechenden Formiat umgesetzt. Aus Pentanalen entstehen hauptsächlich Aldoladdition- und/oder Aldolkondensationsprodukte, die einen geringeren Dampfdruck als die Pentanale aufweisen. Formiate, Folgeprodukte der Pentanale und auch Pentanole sind in der wässrigen Phase löslich.

Mit der Laugenwäsche erfolgt also sowohl eine chemische Umsetzung als auch eine Extraktion der Störkomponenten bzw. deren Folgeprodukte in die wässrige Phase.

Die Laugenwäsche kann in einem oder mehreren Reaktor(en) mit nachgeschalteter Apparatur zur Phasentrennung durchgeführt werden.

Vorzugsweise erfolgt die Laugenbehandlung in einer Extraktionsapparatur. Es können die dem Fachmann bekannten Extraktionsapparate, wie beispielsweise einfache Extraktionskolonnen, Siebbodenkolonnen, Füllkörperkolonnen oder Kolonnen mit bewegten Einbauten eingesetzt werden. Beispiele für Extraktionsapparate mit bewegten Einbauten sind u. a. der Drehscheibenextraktor und die Scheibelkolonne. Ein weiterer Apparat, der insbesondere bei hohen Durchsätzen für die Extraktion eingesetzt wird, ist der sogenannte Mischer-Abscheider-Extraktor (mixer-settler-extractor). Es können auch zwei oder mehrere Extraktoren gleicher oder unterschiedlicher Bauart kombiniert sein.

Da sowohl die chemische Umsetzung als auch eine Extraktion der Störkomponenten bzw. deren Folgeprodukte in die wässrige Phase sehr schnell erfolgt, sind nur wenige theoretische Trennstufen zur ausreichenden Abreinigung des n-Butan/n-Buten-Gemischen erforderlich. Daher erfolgt die Entfernung der Störkomponenten im erfindungsgemäßen Verfahrens besonders bevorzugt in einem einstufigen Mischer-Abscheider-Extraktor.

Als Mischer können beispielsweise Injektoren, Kreiselpumpen, Mammutpumpen, mechanische Rührer oder auch statischer Mischer eingesetzt werden, in denen die organische Phase und die Lauge innig vermischt werden. Im erfindungsgemäßen Verfahren werden bevorzugt statische Mischer oder Mischpumpen, insbesondere Kreiselpumpen, eingesetzt.

Als Abscheider können Abscheider eingesetzt werden, die die Phasentrennung mit alleiniger Ausnutzung der Schwerkraft ermöglichen. Diese sogenannten Schwerkraftabscheider können auch mit Einbauten als koaleszenzfördernde Maßnahme zur Erhöhung der Trennleistung ausgeführt werden. Als Koaleszenzhilfen können z. B. Platten, Füllkörper, Gewebepackungen oder Faserbettabscheidern eingesetzt werden. Schwerkraftabscheider können als liegende Behälter oder als stehende Behälter ausgeführt werden. Alternativ zu Schwerkraftabscheider können zur Flüssig-Flüssig-Trennung aus Separatoren nach dem Prinzip der Zentrifugen eingesetzt werden. Durch Fliehkräfte in einer sich drehenden Trommel wird dabei die schwere Phase abgetrennt. Im erfindungsgemäßen Verfahren werden bevorzugt Schwerkraftabscheider, ausgeführt als liegende Behälter mit Einbauten, eingesetzt.

Soll sowohl ein flüssiger als ein gasförmiger Strom abgereinigt werden, so kann dem Mischer ein Gaswäscher nachgeschaltet werden, in dem der Gasstrom im Gegenstrom zur flüssigen Phase geführt wird. Bevorzugt wird dabei eine Füllkörperkolonne eingesetzt, bei der die flüssige Phase am oberen Kolonnendrittel aufgegeben wird und der Gasstrom im unteren Drittel der Kolonne. Der Abzug der Gasphase erfolgt im oberen Kolonnendrittel, oberhalb der Flüssigkeitsaufgabe, und der Abzug der Flüssigphase erfolgt im unteren Kolonnendrittel, unterhalb der Aufgabe der Flüssigphase. Die flüssige Phase aus dem Gaswäscher wird dann dem Abscheider zugeführt, in dem die Trennung zwischen organischer und wässriger Phase erfolgt.

Die Laugenphase wird im geraden Durchgang oder bevorzugt unter Rückführung (Kreisfahrweise) der Extraktion zugeführt. Die Formiatkonzentrat im Extrakt (Extraktion verlassende Aufnehmerphase) liegt unter 0,2 Massen-%, insbesondere unter 0,1 Massen-%, ganz besonders unter 0,05 Massen-%. Wenn als Lauge Natronlauge verwendet wird, enthält der Extrakt weniger als 0,2 Massen-% Natriumformiat, insbesondere weniger als 0,1 Massen-%, ganz besonders weniger als 0,01 Massen-%. Die Konzentration an Aldoladdition- und/oder Aldolkondensationprodukten der Pentanale im Extrakt liegt unter deren maximalen Löslichkeit in der Lauge. Die Konzentration dieser Pentanalfolgeprodukte im Extrakt liegt unter 0,5 Massen-%, insbesondere unter 0,2 Massen-%. Die Konzentration von Pentanolen im Extrakt liegt unter 0,5 Massen-%. Bei Kreislauffahrweise werden dementsprechend die Konzentrationen der Aufnehmerphase im oberen Kolonnenzulauf derart begrenzt, dass die oben genannten Grenzwerte nicht überschritten werden. Das Durchsatzverhältnis (Masse/Masse) zwischen n-Buten/n-Butan-Phase und Lauge liegt zwischen 30 zu 1 und 2 zu 1, vorzugsweise zwischen 15 zu 1 und 5 zu 1.

Die Extraktion wird bei Temperaturen zwischen 15 und 120 °C, insbesondere zwischen 40 und 90 °C, ganz besonders zwischen 60 und 80 °C. Der Druck in der Extraktion entspricht mindestens dem Wasserpartialdruck der Lauge.

Beim bevorzugten flüssigen Abzug von zumindest einem Teil des Raffinats liegt der Druck in der Extraktion über dem Dampfdruck des n-Butans bei Extraktionstemperatur. Da nach der Extraktion vorzugsweise eine destillative Aufarbeitung erfolgt, ist es zweckmäßig, die Extraktion bei einem höheren Druck als die Destillation durchzuführen, um eine Zwischenverdichtung einzusparen. Bevorzugt liegt der Druck in der Extraktionsapparatur im Bereich von 0,5 bis 5,0 MPa, insbesondere im Bereich von 1,5 bis 3,0 MPa.

Wird zur Extraktion Natronlauge verwendet, kann der Extrakt (beladene Lauge) als Katalysator für die Aldolkondensation von Pentanalen verwendet werden.

Das Raffinat besteht hauptsächlich aus n-Butan. Es enthält 1 bis 30 Massen-% n-Butene, insbesondere 5 bis 15 Massen-%. Der Gehalt an Ameisensäure liegt unter 5 Massen-ppm, insbesondere unter 3 Massen-ppm, ganz besonders unter 1 Massen-ppm. Der Gehalt an Sauerstoff enthaltenden C₅-Komponenten, insbesondere Pentanalen, liegt unter 1 Massen-ppm. Bedingt durch die physikalische Löslichkeit von Wasser in der organischen Phase bei Extraktionstemperatur und - druck enthält das Raffinat geringe Wassermengen. Weiterhin kann das Raffinat C₅-Kohlenwasserstoffe enthalten. Falls auch eine gasförmige Raffinatphase auftritt, besteht diese hauptsächlich aus Synthesegas, das die oben genannten Komponenten entsprechend ihrer Partialdrücke enthält.

Das Raffinat (flüssiger und gasförmiger Anteil) wird destillativ in Kohlenwasserstoffe (hauptsächlich C₄-Kohlenwasserstoffe, Wasser und Gase (hauptsächlich Synthesegas) aufgetrennt. Die Auftrennung erfolgt in mindestens einer Kolonne, bevorzugt in genau einer Kolonne. Die Destillationskolonne ist mit einem Kopfkondensator und einem Phasentrenngefäß versehen. Während der Destillation werden die Brüden (partiell) kondensiert und im Trenngefäß in zwei flüssige Phasen, nämlich eine Kohlenwasserstoffphase und eine Wasserphase sowie gegebenenfalls in eine Gasphase getrennt. Die wässrige Phase und, falls vorhanden, die gasförmige Phase werden ausgeschleust. Als Sumpfprodukt wird ein praktisch wasserfreier und gasfreier Kohlenwasserstoffstrom gewonnen. Die organische Phase aus dem Trenngefäß wird in den oberen Teil der Kolonne zurückgeführt.

Die Destillationskolonne weist vorzugsweise 5 bis 45 theoretische Trennstufen, bevorzugt 8 bis 32 und besonders bevorzugt 12 bis 22 theoretische Trennstufen auf.

Der Zulauf der Kolonne wird vorzugsweise zwischen Stufe 1 und 16 (von oben) zugegeben, besonders bevorzugt zwischen Stufe 1 und 5. Das Verhältnis von zugeführter Zulaufmenge zu Brüdenmenge beträgt, in Abhängigkeit von der realisierten Stufenzahl, der Wasserkonzentration des Kolonnenzulaufs und der erforderlichen Reinheiten des Sumpfproduktes, vorzugsweise kleiner 5, bevorzugt kleiner 1. Der Betriebsdruck der Kolonne kann vorzugsweise zwischen 0,6 und 4,0 MPa(abs) eingestellt werden, bevorzugt zwischen 1,0 und 3,0 MPa(abs), besonders bevorzugt zwischen 1,5 und 2,5 MPa(abs).

Die aus dem Trenngefäß abgezogene Gasphase besteht hauptsächlich aus Synthesegas, das Kohlenwasserstoffe (hauptsächlich C₄-Kohlenwasserstoffe) entsprechend deren Partialdrücke und Wasser enthält. Dieses Gasgemisch kann teilweise in eine C₄-Hydroformylierungsanlage (zurück)gefahren werden. Es kann als Heizgas, Rohstoff für eine Synthesegasanlage oder zur Gewinnung von Wasserstoff genutzt werden.

Das Sumpfprodukt der Destillationskolonne besteht zu über 99,9 %, insbesondere zu über 99,95 % aus Kohlenwasserstoffen. Der Wassergehalt liegt unter 20 ppm, bevorzugt unter10 ppm, besonders bevorzugt unter 1 ppm.

Die Hydrierung erfolgt erfindungsgemäß in Gegenwart von Wasserstoff. Wasserstoff wird in überstöchiometrischer Menge eingesetzt. Vorzugsweise beträgt der Wasserstoffüberschuss 5 bis 20 %.

Die vollständige Hydrierung der Olefine im Sumpfprodukt, katalytisch mit Wasserstoff, kann kontinuierlich oder diskontinuierlich in der Gas- oder Flüssigphase durchgeführt werden. Technisch wird die Hydrierung gemäß dem Stand der Technik vorzugsweise an Festbettkatalysatoren in der Flüssigphase vorgenommen.

Als Katalysatoren können prinzipiell alle Katalysatoren eingesetzt werden, die aus der Literatur für die Hydrierung olefinischer Doppelbindung bekannt sind. Dies sind insbesondere Katalysatoren, die als hydrieraktive Metalle Nickel, Palladium oder Platin aufweisen.

Für die Hydrierung werden bevorzugt Palladium-Trägerkatalysatoren eingesetzt. Es werden vorzugsweise Trägerkatalysatoren mit 0,5 bis 1,0 % Massen-% Palladium auf oberflächenreichen γ-Aluminiumoxiden verwendet.

Die Hydrierung in der flüssigen Phase wird im Temperaturbereich von 20 bis 140 °C, insbesondere im Temperaturbereich von 40 bis 100, ganz besonders im Temperaturbereich von 30 bis 60 °C durchgeführt. Der Druck in der Hydrierapparatur liegt über dem Dampfdruck des n-Butans. Vorzugsweise liegt der Druck zwischen 0,6 und 3 MPa, insbesondere zwischen 1 und 2 MPa.

Die Hydrierung kann in einem oder bevorzugt in mehreren Reaktor(en) durchgeführt werden. Wird die Hydrierung in zwei hintereinander geschalteten Reaktoren vorgenommen, wird der erste bevorzugt in Schlaufenfahrweise und der zweite im geraden Durchgang betrieben.

Der Hydrieraustrag enthält weniger als 1000 ppm Massen-ppm Olefine, bevorzugt weniger als 500 Massen-ppm Olefine, besonders bevorzugt weniger als 200 Massen-ppm.

Optional kann aus dem Hydrieraustrag in einer weiteren Kolonne geringe Mengen an C₅-Kohlenwasserstoffen und gelöster Wasserstoff durch Destillation abgetrennt werden.

Die Erfindung soll nun anhand von Anlagen-Schaltbildern erläutert werden. Hierfür zeigen:
- Fig. 1:: Anlage zur erfindungsgemäßen Laugenextraktion zur Behandlung von flüssigen Einsatzgemischen;
- Fig. 2:: Anlage zur erfindungsgemäßen Laugenextraktion zur Behandlung von zweiphasigen Einsatzgemischen;
- Fig. 3:: Anlage zur destillativen Entwässerung;
- Fig. 4:: Anlage zur Hydrierung des Olefinstroms und zur destillativen Abtrennung von C₅-Kohlenwasserstoffen.

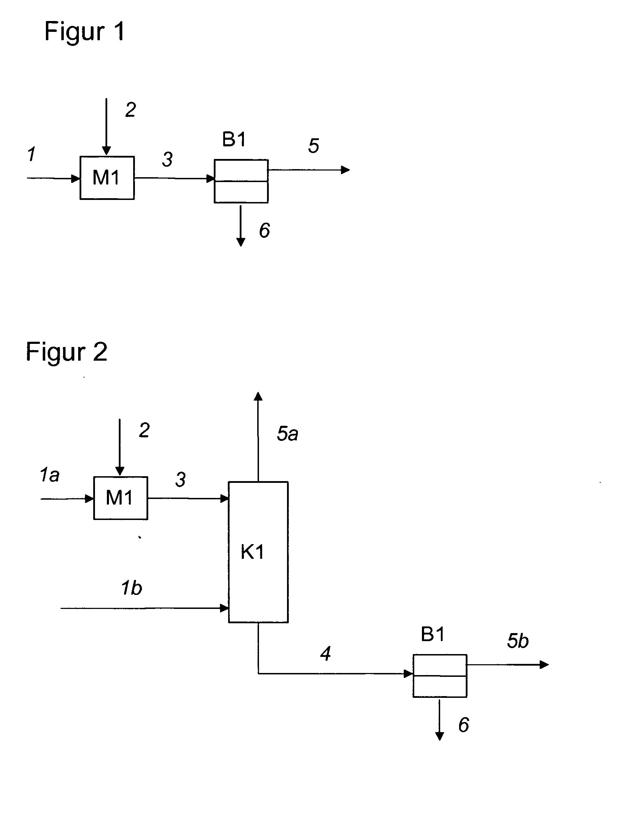

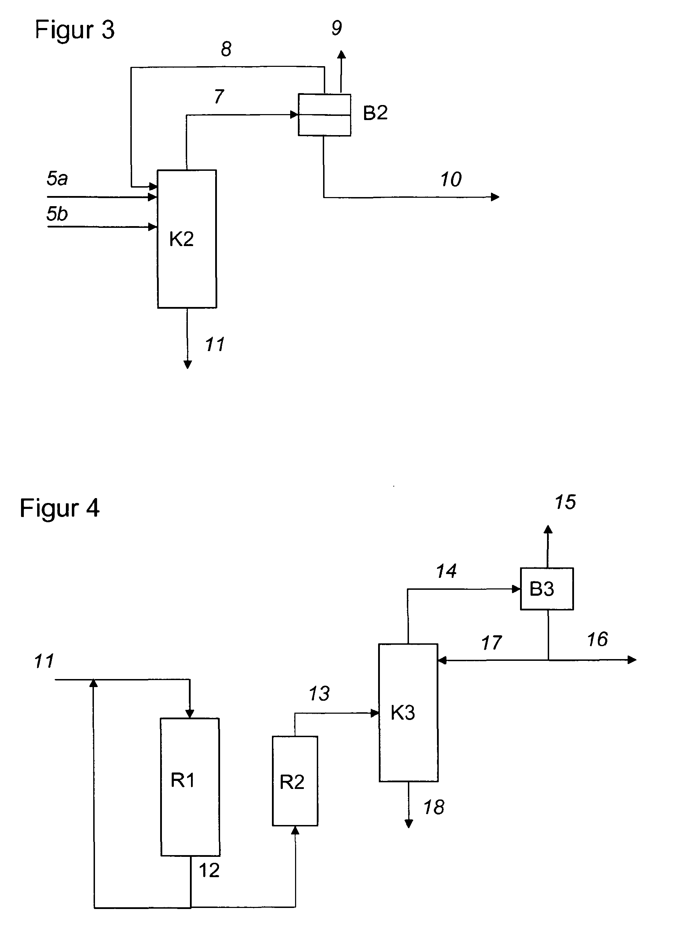

In Figur 1 ist die erfindungsgemäße Extraktion dargestellt für den Fall, dass das Einsatzgemisch ausschließlich flüssig vorliegt. Das Einsatzgemisch, enthaltend Kohlenmonoxid, n-Butan, n-Butene, geringe Mengen an Ameisensäure und sauerstoffhaltige C₅-Verbindungen, wird dem Mischer M1 zugeführt und hier mit der Extraktionslauge (2) vermischt. Die zweiphasige Mischung (3) wird dem Abscheider B1 zugeführt. In dem Abscheider B1 erfolgt die Phasentrennung, wobei der Extrakt (6) als wässrige Phase, beladen mit den abzutrennenden Verunreinigungen, ausgeschleust wird. Ein Teil des Extraktes (6) kann gegebenenfalls als Kreislaufstrom in den Mischer zurückgeführt werden (nicht eingezeichnet). Das flüssige Raffinat (5) (hauptsächlich ein n-Butan/n-Butene-Gemisch) wird der destillativen Trocknung K2 (in Figur 3) zugeführt.

Figur 2 zeigt eine Verfahrensvariante der Extraktion, bei der der Einsatzstoff aus einem flüssigen Teilstrom (1 a) und einem gasförmigen Teilstrom (1 b) besteht. Der gasförmige Strom enthält neben n-Butan, n-Butenen, geringe Mengen an . Ameisensäure und gegebenenfalls sauerstoffhaltige C₅-Verbindungen gemäß der Partialdrücke der Komponenten überwiegend Synthesegas, also Kohlenmonoxid und Wasserstoff. Wie in Figur 1 wird der flüssige Einsatzstrom (1 a) dem Mischer M1 zugeführt und hier mit der Extraktionslauge (2) vermischt. Die zweiphasige Mischung (3) wird dem Wäscher K1 zugeführt. In dem Wäscher wird der gasförmige Einsatzstrom (1 b) im Gegenstrom mit dem flüssigen Gemisch (3) gewaschen bzw. extrahiert. Das gasförmige Extrakt (5a), weitestgehend frei von den störenden Verunreinigungen, wird im oberen Teil des Wäschers K1 abgezogen und der destillativen Trocknung K2 (in Figur 3) zugeführt. Der zweiphasige flüssige Strom (4) wird im unteren Teil des Wäschers K1 abgezogen und dem Abscheider B1 zugeführt. In dem Abscheider B1 erfolgt wieder die Phasentrennung, wobei die das Extrakt (6) als wässrige Phase, beladen mit den abzutrennenden Verunreinigungen, ausgeschleust wird. Ein Teil des Extraktes (6) kann ggf. wieder als Kreislaufstrom in den Mischer zurückgeführt werden (nicht eingezeichnet). Das flüssige Raffinat (5b) wird wieder der destillativen Trocknung K2 (in Figur 3) zugeführt. Der untere Teil des Wäscher K1 kann optional auch so ausgestaltet werden, dass der Abscheider B1 in den Wäscher K1 integriert wird und die Phasentrennung der flüssigen Ströme (5b) und (6) im Wäscher erfolgt (nicht eingezeichnet).

In Figur 3 ist die destillative Trocknung des Raffinats in der Kolonne K2 dargestellt. Der flüssige Raffinatstrom (5) wird bzw. der flüssige Raffinatstrom (5b) und der gasförmige Raffinatstrom (5a) werden in den oberen Teil der Destillationskolonne K2 eingeleitet. Der Brüden (7) der Kolonne K2 wird (partiell) kondensiert (Kondensator nicht eingezeichnet) und das dabei entstehende Kondensat wird im Behälter B2 in eine Gasphase (9), eine flüssige Wasserphase (10) und eine flüssige organische Phase (8) getrennt. Die Gasphase (9) (hauptsächlich Synthesegas) und die Wasserphase (10) werden ausgeschleust, während die organische Phase (8) wieder in den oberen Teil der Kolonne K2 zurückgeführt wird. Als Sumpfprodukt (11) der Kolonne K2 wird ein praktisch wasserfreies Kohlenwasserstoffgemisch abgezogen und der Hydrierung (in Figur 4) zugeführt.

In Figur 4 sind die Hydrierung des Olefinstroms sowie die destillative Abtrennung der C₅-Kohlenwasserstoffe dargestellt. Die Olefine im Strom (11) werden in den Reaktoren R1 und R2 hydriert. (Die Wasserstoffzufuhr zu den Hydrierreaktoren und die Abtrennung von Abgas sind in Figur 4 nicht eingezeichnet.) Der Reaktoraustrag (13) wird in die Destillationskolonne K3 eingespeist. Der Brüden (14) der Kolonne K3 wird kondensiert (Kondensator nicht eingezeichnet) und das dabei anfallende Kondensat wird im Behälter B3 in eine Gasphase und eine Flüssigphase getrennt. Die Gasphase (15) besteht überwiegend aus Wasserstoff und wird ausgeschleust. Die Flüssigphase besteht aus nahezu reinem n-Butan. Ein Teil der Flüssigphase wird mit Strom (16) als Zielprodukt abgezogen, während der andere Teil (17) als Rückfluss in die Kolonne K3 gefahren wird. Als Sumpfprodukt (18) fallen geringe Mengen an C₅-Kohlenwasserstoffen und ggf. Spuren anderer Hochsieder an.

Die folgenden Beispiele sollen die Erfindung erläutern, ohne die Anwendungsbreite einzuschränken, die sich aus der Beschreibung und den Patentansprüchen ergibt.

### Beispiel 1: Entfernung von Ameisensäure durch Laugeextraktion

### Versuchsanlage:

Die Untersuchungen zur Entfernung von Ameisensäurespuren durch Laugeextraktion bzw. -wäsche aus einem wurden in einer Laborversuchsanlage durchgeführt, die im Wesentlichen aus einer Dosier-, Extraktionseinheit bestand. Das Kernstück der Laboranlage war die Extraktionseinheit in Form eines Doppelmantel-Reaktors aus Edelstahl von 1,3 m Länge und 25 mm Durchmesser, der vollständig mit 3 mm Glasperlen befüllt wurde. Um die Vermischung der organischen Phase mit der wässrigen Natronlauge zu verbessern, wurde der Reaktor mit einem Kreislauf versehen. Für die Kreislaufumwälzung sorgte eine vor dem Reaktor installierte Reaktionsmischpumpe. Die Natronlauge als Extraktions- bzw. Waschmedium wurde direkt in die Reaktionsmischpumpe zudosiert. Für die Trennung der organischen und der wässrigen Phase nach dem Reaktor wurde ein 2 Liter Trennbehälter installiert.

Als Edukt wurde ein Butan/Buten-Gemisch mir 10 Massen-% Butenen verwendet, das mit 500 ppm Ameisensäure versetzte wurde. Das Einsatzgemisch wurde aus einer 30 I Druckvorlage entnommen und mit einer Lewa-Pumpe in den Reaktor geleitet. Kurz vor dem Reaktor wurde dem Einsatzgemisch aus der Leitung CO über ein Massendurchflussmesser beigemischt. Nach der Extraktionseinheit wurde das Gemisch in ein 50 I Produktfass geleitet. Ein Teilstrom des Gemisches wurde in den 2 I Trennbehälter geführt und analysiert.

### Versuchsdurchführung :

n-Butan/ Buten-Gemisch mit einem Ameisensäuregehalt von 500 ppm wurde mit einem Durchsatz von 650 ml/h aus der 30 I Vorlage in Gegenwart von Natronlauge und CO mittels einer Reaktionspumpe bei drei Temperaturen 40, 60 und 80 °C über die Extraktionseinheit (Waschkolonne) durchgeleitet.

Die als Waschmedium verwendete 25%-ige Natronlauge (70 ml/h) und das CO (500 Nml/h) wurden direkt der Reaktionspumpe zugeführt. Nach der Phasentrennung wurde die organische Phase analysiert und der Ameisensäurerestgehalt bestimmt. Bei zwei Versuchen wurden zusätzlich die H₂O-Gehalte in der organische Phase ermittelt.

Das Volumenverhältnis der organischen Phase zu wässriger Phase lag bei allen Versuchen bei rd.10 zu 1.

### Ergebnisse :

Die Ergebnisse der Versuche zur Ameisensäureentfernung durch Wäsche sind in Tabelle 1 zusammengestellt.

**Tabelle1: Versuchsergebnisse zur Entfernung von Ameisensäure mittels Laugeextraktion.**

| **Versuch Nr.** | | 1 | 2 | 3 | 4 |
|---|---|---|---|---|---|
| | Einheit | | | | |

| **Edukte :** | | | | | |
|---|---|---|---|---|---|
| | | | | | |
| n-Butan/Buten | ml/h | 650 | 650 | 650 | 650 |
| HCOOH in C4-KW | ppm | 500 | 500 | 500 | 500 |
| CO | ml/h | 500 | 500 | 500 | 500 |
| NaOH (25%ig) | ml/h | 70 | 70 | 70 | 70 |
| | | | | | |
| Temperatur | °C | 42 | 60 | 70 | 80 |
| Druck | bar | 20 | 20 | 20 | 20 |
| Kreislauf | I/h | 150 | 150 | 150 | 150 |
| | | | | | |

| **Analyse :** | | | | | |
|---|---|---|---|---|---|
| | | | | | |
| HCOOH | ppm | < 5 ppm | < 5 ppm | < 5 ppm | < 10 ppm |
| pH-Wert vor | | 13,0 | 13,0 | 13,0 | 13,0 |
| pH-Wert nach | | 12,9 | 13,0 | 13,0 | 13,0 |
| H2O in C₄-KW | ppm | 50 | 45 | 55 | 60 |

Wie aus der Tabelle 1 (Versuche 1 bis 4) zu entnehmen ist, wird die Ameisensäure in der organischen Phase durch die NaOH-Wäsche im Temperaturbereich zwischen 40 und 80 °C praktisch vollständig entfernt. Die Ameisensäuregehalte von 500 ppm im Butan/Butengemisch gehen nach der Wäsche auf Restgehalte < 5 ppm (Nachweisgrenze der verwendeten Analytikmethode) zurück

### Beispiel 2: Hydrierung von n-Butenen in Gegenwart von Ameisensäure

Ein Raffinat III aus dem C₄-Strang der Anmelderin, das neben den C₄-Paraffinen n-Butan (72,3 Massen-%) und Isobutan (0,1 Massen-%) rd. 27, 6 Massen-% n-Butene enthielt, wurde mit 100 ppm Ameisensäure versetzt und anschließend in einem Labor-Kreislaufreaktor hydriert. Als Katalysator wurde ein Pd-haltiger Trägerkatalysator H14814 von Degussa mit 0,5 Massen-% Palladium auf γ-Aluminiumoxid verwendet.

Es wurden stündlich 200 ml Edukt bei einer Temperatur von 45 °C und einem Druck von 12 bar über 5 g Katalysator durchgeleitet, entsprechend einem LHSV-Wert von rd. 27 h⁻¹. Der LHSV-Wert (Liquid-Hourly-Space-Velocity) dient als Maß für die Katalysatorbelastung. Zu Beginn des Versuches nach rd. 8 Stunden wurde ein n-Buten-Umsatz von rd. 97,1 % ermittelt. Mit der Fortdauer der kontinuierlichen Hydrierung nahm der Umsatz in den ersten 100 Stunden allmählich ab. Nach rd. 150 Stunden Versuchzeit wurde ein stationärer Zustand mit einem Umsatz von rd. 86,7 % erreicht. Diese Umsatzwerte blieben bis zu Abschluss des Versuches nach 500 Stunden konstant.

### Beispiel 3 Hydrierung von n-Butenen ohne Ameisensäure

Ein Raffinat III, gleicher Zusammensetzung wie im Beispiel 2, allerdings ohne Spuren an Ameisensäure, wurde in einem Labor-Kreislaufreaktor unter gleichen Reaktionsbedingungen (gleicher Katalysator, Durchsatz, Temperatur und Druck) wie im Beispiel 2 hydriert.

Unter den gewählten Bedingungen wurde nach einer Anfahrphase von rd. 50 Stunden Versuchzeit in stationärem Zustand ein Umsatz von 97,8 %, entsprechend einem Restgehalt an n-Butenen von 0,6 Massen-%, ermittelt. Dieser hohe Umsatz blieb bis zu Abschluss des Versuches nach 500 Stunden konstant.

Ein Vergleich der n-Butene-Umsätze mit und ohne Ameisensäure im Edukt zeigt, dass in Gegenwart von Ameisensäure ein deutlicher Rückgang des Umsatzes auf einen konstanten Wert festzustellen ist.

## Patentansprüche

1. Verfahren zur Herstellung von geruchsarmen n-Butan durch katalytische Hydrierung eines Einsatzgemisches,
a) wobei das Einsatzgemisch neben n-Butan, n-Buten und Kohlenmonoxid mindestens eine der folgenden Komponenten enthält:
- bis zu 1 Massen-% Ameisensäure,
- bis zu 1 Massen-% Pentanale,
- bis zu 0,5 Massen-% Pentanole,
b) wobei das Einsatzgemisch im Temperaturbereich von 15 bis 120 °C mit einer wässrigen Lösung eines Alkalimetall- oder Erdalkalimetallhydroxids im Konzentrationsbereich von 0,5 bis 30 Massen-% behandelt wird,
c) und wobei das Einsatzgemisch anschließend der katalytischen Hydrierung unterzogen wird.

2. Verfahren gemäß Anspruch 1,
**dadurch gekennzeichnet,**
**dass** es sich bei der wässrigen Lösung um Natronlauge im Konzentrationsbereich von 0,5 bis 2,5 Massen-% handelt.

3. Verfahren gemäß Anspruch 1 oder 2,
**dadurch gekennzeichnet,**
**dass** die Behandlung mit der wässrigen Lösung in einem Druckbereich von 0,5 bis 5 MPa durchgeführt wird.

4. Verfahren nach Anspruch 1, 2 oder 3,
**dadurch gekennzeichnet,**
**dass** der Anteil des Kohlemonoxids am Einsatzgemisch 1 bis 10, vorzugsweise 2 bis 7 Massenprozent beträgt.

5. Verfahren nach mindestens einem der vorhergehenden Ansprüche,
**dadurch gekennzeichnet,**
**dass** das Einsatzgemisch Wasserstoff enthält.

6. Verfahren nach mindestens einem der vorhergehenden Ansprüche,
**dadurch gekennzeichnet,**
**dass** die Extraktion in einer Mischer-Abscheider-Einheit durchgeführt wird.

7. Verfahren nach Anspruch 6,
**dadurch gekennzeichnet,**
**dass** als Mischer ein statischer Mischer eingesetzt wird.

8. Verfahren nach Anspruch 6,
**dadurch gekennzeichnet,**
**dass** als Mischer eine Pumpe, insbesondere eine Kreiselpumpe, eingesetzt wird.

9. Verfahren gemäß mindestens einem der vorigen Ansprüche,
**dadurch gekennzeichnet,**
**dass** die Extraktion in einer Extraktionskolonne durchgeführt wird.

10. Verfahren nach mindestens einem der vorigen Ansprüche,
**dadurch gekennzeichnet,**
**dass** das extrahierte n-Butan/n-Buten-Gemisch ganz oder teilweise als Flüssigkeit aus der Extraktionsapparatur abgezogen wird.

11. Verfahren nach mindestens einem der vorigen Ansprüche,
**dadurch gekennzeichnet;**
**dass** die Hydrierung in flüssiger Phase an einem Palladiumkatalysator im Temperaturbereich von 20 bis 140 °C und im Druckbereich von 0,6 bis 3 MPa durchgeführt wird.

12. Verfahren nach mindestens einem der vorigen Ansprüche,
**dadurch gekennzeichnet,**
**dass** das extrahierte n-Butan/n-Butengemisch destillativ entwässert wird.

13. Verfahren nach mindestens einem der Ansprüche 1 bis 8,
**dadurch gekennzeichnet,**
**dass** der Hydrieraustrag destillativ entwässert wird.

14. Verfahren nach mindestens einem der Ansprüche 1 bis 10,
**dadurch gekennzeichnet,**
**dass** C₅-Kohlenwasserstoffe durch Destillation abgetrennt werden.

15. Verfahren nach mindestens einem der Ansprüche 1 bis 11,
**dadurch gekennzeichnet,**
**dass** als Einsatzstoffgemisch ein Strom aus einer C₄-Hydroformylierungsanlage eingesetzt wird.

## Claims

1. Process for preparing low-odour n-butane by catalytically hydrogenating a starting mixture,
a) wherein the starting mixture comprises, in addition to n-butane, n-butene and carbon monoxide, at least one of the following components:
- up to 1% by mass of formic acid,
- up to 1% by mass of pentanals,
- up to 0.5% by mass of pentanols,
b) wherein the starting mixture is treated within the temperature range from 15 to 120°C with an aqueous solution of an alkali metal hydroxide or alkaline earth metal hydroxide within the concentration range from 0.5 to 30% by mass,
c) and wherein the starting mixture is subsequently subjected to catalytic hydrogenation.

2. Process according to Claim 1,
**characterized in that**
the aqueous solution is sodium hydroxide solution within the concentration range from 0.5 to 2.5% by mass.

3. Process according to Claim 1 or 2,
**characterized in that**
the treatment with the aqueous solution is performed within a pressure range from 0.5 to 5 MPa.

4. Process according to Claim 1, 2 or 3,
**characterized in that**
the proportion of carbon monoxide in the starting mixture is 1 to 10 and preferably 2 to 7% by mass.

5. Process according to at least one of the preceding claims,
**characterized in that**
the starting mixture comprises hydrogen.

6. Process according to at least one of the preceding claims,
**characterized in that**
the extraction is performed in a mixer-settler unit.

7. Process according to Claim 6,
**characterized in that**
the mixer used is a static mixer.

8. Process according to Claim 6,
**characterized in that**
the mixer used is a pump, especially a centrifugal pump.

9. Process according to at least one of the preceding claims,
**characterized in that**
the extraction is performed in an extraction column.

10. Process according to at least one of the preceding claims,
**characterized in that**
the extracted n-butane/n-butene mixture is drawn off from the extraction apparatus entirely or partly in liquid form.

11. Process according to at least one of the preceding claims,
**characterized in that**
the hydrogenation is performed in the liquid phase over a palladium catalyst within the temperature range from 20 to 140°C and within the pressure range from 0.6 to 3 MPa.

12. Process according to at least one of the preceding claims,
**characterized in that**
the n-butane/n-butene mixture extracted is dewatered by distillation.

13. Process according to at least one of Claims 1 to 8,
**characterized in that**
the hydrogenation output is dewatered by distillation.

14. Process according to at least one of Claims 1 to 10,
**characterized in that**
C₅ hydrocarbons are removed by distillation.

15. Process according to at least one of Claims 1 to 11,
**characterized in that**
the starting mixture used is a stream from a C₄ hydroformylation plant.

## Revendications

1. Procédé pour la préparation de n-butane peu odorant par hydrogénation catalytique d'un mélange de départ,
a) le mélange de départ contenant, outre du n-butane, du n-butène et du monoxyde de carbone, au moins un des composants suivants :
- jusqu'à 1% en masse d'acide formique,
- jusqu'à 1% en masse de pentanals,
- jusqu'à 0,5% en masse de pentanols,
b) le mélange de départ étant traité dans une plage de température de 15 à 120°C avec une solution aqueuse d'un hydroxyde de métal alcalin ou alcalino-terreux dans la plage de concentration de 0,5 à 30% en masse,
c) et le mélange de départ étant ensuite soumis à l'hydrogénation catalytique.

2. Procédé selon la revendication 1, **caractérisé en ce qu'**il s'agit, pour la solution aqueuse, de lessive de soude caustique dans la plage de concentration de 0,5 à 2,5% en masse.

3. Procédé selon la revendication 1 ou 2, **caractérisé en ce que** le traitement par la solution aqueuse est réalisé dans une plage de pression de 0,5 à 5 MPa.

4. Procédé selon la revendication 1, 2 ou 3, **caractérisé en ce que** la proportion de monoxyde de carbone dans le mélange de départ est de 1 à 10, de préférence de 2 à 7% en masse.

5. Procédé selon au moins l'une quelconque des revendications susmentionnées, **caractérisé en ce que** le mélange de départ contient de l'hydrogène.

6. Procédé selon au moins l'une quelconque des revendications susmentionnées, **caractérisé en ce que** l'extraction est réalisée dans une unité mélangeur-séparateur.

7. Procédé selon la revendication 6, **caractérisé en ce qu'**on utilise un mélangeur statique en tant que mélangeur.

8. Procédé selon la revendication 6, **caractérisé en ce qu'**on utilise, comme mélangeur, une pompe, en particulier une pompe centrifuge.

9. Procédé selon au moins l'une quelconque des revendications précédentes, **caractérisé en ce que** l'extraction est réalisée dans une colonne d'extraction.

10. Procédé selon au moins l'une quelconque des revendications précédentes, **caractérisé en ce que** le mélange n-butane/n-butène extrait est soutiré totalement ou partiellement sous forme de liquide de l'appareillage d'extraction.

11. Procédé selon au moins l'une quelconque des revendications précédentes, **caractérisé en ce que** l'hydrogénation est réalisée en phase liquide sur un catalyseur de palladium dans une plage de température de 20 à 140°C et une plage de pression de 0,6 à 3 MPa.

12. Procédé selon au moins l'une quelconque des revendications précédentes, **caractérisé en ce que** le mélange n-butane/n-butène extrait est déshydraté par distillation.

13. Procédé selon au moins l'une quelconque des revendications 1 à 8, **caractérisé en ce que** le produit sortant de l'hydrogénation est déshydraté par distillation.

14. Procédé selon au moins l'une quelconque des revendications 1 à 10, **caractérisé en ce que** les hydrocarbures en C₅ sont séparés par distillation.

15. Procédé selon au moins l'une quelconque des revendications 1 à 11, **caractérisé en ce qu'**on utilise, comme mélange de substances de départ, un flux sortant d'une installation d'hydroformylation de substances en C₄.
